(19) Europäisches Patentamt
European Patent Office
Office européen des brevets

(11) EP 0 882 021 B1

(12) **EUROPÄISCHE PATENTSCHRIFT**

(45) Veröffentlichungstag und Bekanntmachung des Hinweises auf die Patenterteilung:
**05.03.2003 Patentblatt 2003/10**

(21) Anmeldenummer: **97904354.4**

(22) Anmeldetag: **30.01.1997**

(51) Int Cl.[7]: **C07D 221/12**, A61K 31/47

(86) Internationale Anmeldenummer:
**PCT/EP97/00402**

(87) Internationale Veröffentlichungsnummer:
**WO 97/028131 (07.08.1997 Gazette 1997/34)**

(54) **NEUE PHENANTHRIDINE**
NEW PHENANTHRIDINES
NOUVELLES PHENANTHRIDINES

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **31.01.1996 DE 19603321**
**08.02.1996 EP 96101791**

(43) Veröffentlichungstag der Anmeldung:
**09.12.1998 Patentblatt 1998/50**

(73) Patentinhaber: **ALTANA Pharma AG**
**78467 Konstanz (DE)**

(72) Erfinder:
- **AMSCHLER, Hermann (DE)**
- **FLOCKERZI, Dieter D-78476 Allensbach (DE)**
- **ULRICH, Wolf-Rüdiger D-78464 Konstanz (DE)**
- **BÄR, Thomas D-78479 Reichenau (DE)**
- **MARTIN, Thomas D-78462 Konstanz (DE)**
- **SCHUDT, Christian D-78462 Konstanz (DE)**
- **HATZELMANN, Armin D-78467 Konstanz (DE)**
- **BEUME, Rolf D-78465 Konstanz (DE)**
- **HÄFNER, Dietrich D-78464 Konstanz (DE)**
- **BOSS, Hildegard D-78476 Allensbach (DE)**
- **KLEY, Hans-Peter D-78476 Allensbach (DE)**
- **GOEBEL, Karl-Josef D-78315 Radolfzell (DE)**
- **SCHMIDT, Beate D-78476 Allensbach (DE)**

(56) Entgegenhaltungen:
**EP-A- 0 045 171** **FR-A- 2 144 609**

- **JOURNAL OF LIQUID CHROMATOGRAPHY, Bd. 13, Nr. 3, 1990, Seiten 543-555, XP000196246 MASATAKA MORIYASU ET AL.: "A semicontinuous assay of inhibition of Cyclic-AMP Phosphodiesterase by Benzo[c]phenanthridine alkaloids"**
- **BERICHTE DER DEUTSCHEN CHEMISCHEN GESELLSCHAFT, Bd. 72, Nr. 4, 1939, Seiten 675-678, XP000196051 SHIGEHIKO SUGASAWA ET AL.: "Synthese partiell hydrierter Phenanthridin-Derivate" in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

### Anwendungsgebiet der Erfindung

Die Erfindung betrifft neue 6-Phenylphenanthridine, die in der pharmazeutischen Industrie zur Herstellung von Medikamenten verwendet werden.

### Bekannter technischer Hintergrund

In Chem. Ber. **1939**, 72, 675-677, J. Chem. Soc., **1956**, 4280-4283 und J. Chem. Soc. (C), **1971**, 1805-1808 wird die Synthese von 6-Phenylphenanthridinen beschrieben.

### Beschreibung der Erfindung

Es wurde nun gefunden, daß die nachfolgend näher beschriebenen neuen Phenanthridine überraschende und besonders vorteilhafte Eigenschaften besitzen.

Gegenstand der Erfindung sind somit Verbindungen der Formel I (siehe beigefügtes Formelblatt). worin

R1 Hydroxy, 1-4C-Alkoxy, 3-7C-Cycloalkoxy oder 3-7C-Cycloalkylmethoxy oder ganz oder teilweise durch Fluor substituiertes 1-4C-Alkoxy bedeutet,

R2 Hydroxy, 14C-Alkoxy, 3-7C-Cycloalkoxy oder 3-7C-Cycloalkylmethoxy oder ganz oder teilweise durch Fluor substituiertes 1-4C-Alkoxy bedeutet,

oder worin

R1 und R2 gemeinsam eine 1-2C-Alkylendioxygruppe bedeuten,
R3 Wasserstoff oder 1-4C-Alkyl bedeutet,
R31 Wasserstoff oder 1-4C-Alkyl bedeutet,

oder worin

R3 und R31 gemeinsam eine 1-4C-Alkylengruppe bedeuten,
R4 Wasserstoff oder 1-4C-Alkyl bedeutet,
R5 Wasserstoff bedeutet,
R51 Wasserstoff bedeutet,

oder worin

R5 und R51 gemeinsam eine zusätzliche Bindung darstellen,
R6 einen durch R7 substituierten Phenylrest darstellt, wobei
R7 COOR71 oder CON(R72)R73 bedeutet und

R71 Wasserstoff, 1-7C-Alkyl, 3-7C-Cycloalkyl oder 3-7C-Cycloalkylmethyl bedeutet und
R72 und R73 unabhängig voneinander

Wasserstoff, 1-7C-Alkyl, 3-7C-Cycloalkyl oder 3-7C-Cycloalkylmethyl bedeuten, sowie die Salze dieser Verbindungen.

1-4C-Alkoxy steht für Reste, die neben dem Sauerstoffatom einen geradkettigen oder verzweigten Alkylrest mit 1 bis 4 Kohlenstoffatomen enthalten. Beispielsweise seien genannt der Butoxy-, iso-Butoxy-, sec.-Butoxy-, tert. -Butoxy-, Propoxy-, Isopropoxy- und bevorzugt der Ethoxy- und Methoxyrest.

3-7C-Cycloalkoxy steht beispielsweise für Cyclopropyloxy, Cyclobutyloxy, Cyclopentyloxy, Cyclohexyloxy und Cycloheptyloxy, wovon Cyclopropyloxy, Cyclobutyloxy und Cyclopentyloxy bevorzugt sind.

3-7C-Cycloalkylmethoxy steht beispielsweise für Cyclopropylmethoxy, Cyclobutylmethoxy, Cyclopentylmethoxy, Cyclohexylmethoxy und Cycloheptylmethoxy, wovon Cyclopropylmethoxy, Cyclobutylmethoxy und Cyclopentylmethoxy bevorzugt sind.

Als ganz oder teilweise durch Fluor substituiertes 1-4C-Alkoxy seien beispielsweise der 1,2,2-Trifluorethoxy-, der 2,2,3,3,3-Pentafluorpropoxy-, der Perfluorethoxy-, der 1,1,2,2-Tetrafluorethoxy-, der Trifluomethoxy-, insbesondere der 2,2,2-Trifluorethoxy- und bevorzugt der Difluormethoxyrest genannt.

1-4C-Alkyl steht für einen geradkettigen oder verzweigten Alkylrest mit 1 bis 4 Kohlenstoffatomen. Beispielsweise seien genannt der Butyl-, iso-Butyl-, sec.-Butyl-, tert.-Butyl-, Propyl-, Isopropyl- und bevorzugt der Ethyl- und Methylrest.

1-2C-Alkylendioxy steht beispielsweise für den Methylendioxy-(-O-$CH_2$-O-) und den Ethylendioxyrest (-O-$CH_2$-$CH_2$-O-).

Haben R3 und R31 gemeinsam die Bedeutung 1-4C-Alkylen, so sind die Positionen 1 und 4 in Verbindungen der Formel I durch eine 1-4C-Alkylenbrücke miteinander verknüpft, wobei 1-4C-Alkylen für geradkettige oder verzweigte Alkylenreste mit 1 bis 4 Kohlenstoffatomen steht. Beispielsweise seien die Reste Methylen (-$CH_2$), Ethylen (-$CH_2$-$CH_2$-), Trimethylen (-$CH_2CH_2CH_2$), 1,2-Dimethylethylen [-CH($CH_3$)-CH($CH_3$)-] und Isopropyliden [-C($CH_3$)$_2$-] genannt.

Wenn R5 und R51 gemeinsam eine zusätzliche Bindung bedeuten, dann sind die Kohlenstoffatome in den Positionen 2 und 3 der Verbindungen der Formel I über eine Doppelbindung miteinander verknüpft.

1-7C-Alkyl steht für geradkettige oder verzweigte Alkylreste mit 1 bis 7 Kohlenstoffatomen. Beispielsweise seien genannt der Heptyl-, Isoheptyl-(2-Methylhexyl-), Hexyl-, Isohexyl- (2-Methylpentyl-), Neohexyl- (2,2-Dimethylbutyl-), Pentyl-, Isopentyl- (3-Methylbutyl-), Neopentyl- (2,2-Dimethylpropyl-), Butyl-, iso-Butyl-, sec.-Butyl-, tert.-Butyl-, Propyl-, Isopropyl-, Ethyl- und der Methylrest.

3-7C-Cycloalkyl steht für den Cyclopropyl-, Cyclobutyl-, Cyclopentyl-, Cyclohexyl- und Cycloheptylrest. Bevorzugt seien die 3-5C-Cycloalkylreste Cyclopropyl, Cyclobutyl und Cyclopentyl genannt.

3-7C-Cycloalkylmethyl steht für einen Methylrest, der durch einen der vorstehend genannten 3-7C-Cycloalkylreste substituiert ist. Bevorzugt seien die 3-5C-Cycloalkylmethylreste Cyclopropylmethyl, Cyclobutylmethyl und Cyclopentylmethyl genannt.

Der Substituent R7 kann in jeder geeigneten Position am Phenylring angebunden sein. Insbesondere bevorzugt ist die Anbindung des Substituenten R7 in 4-Position des Phenylrings.

Als beispielhafte durch R7 substituierte Phenylreste seien genannt 4-Carboxyphenyl, 3-Carboxyphenyl, 4-Methoxycarbonylphenyl, 3-Methoxycarbonylphenyl, 2-Methoxycarbonylphenyl, 4-Ethoxycarbonylphenyl, 3-Ethoxycarbonylphenyl, 2-Ethoxycarbonylphenyl, 4-(N-Methylaminocarbonyl)phenyl, 3-(N-Methylaminocarbonyl)phenyl, 4-(N, N-Dimethylaminocarbonyl)phenyl, 4-Carbamoylphenyl und 3-Carbamoylphenyl.

Hervorzuhebende Verbindungen der Formel I sind solche, worin

R1 1-4C-Alkoxy, 3-7C-Cycloalkoxy oder 3-7C-Cycloalkylmethoxy oder ganz oder teilweise durch Fluor substituiertes 1-2C-Alkoxy bedeutet,
R2 1-4C-Alkoxy, 3-7C-Cycloalkoxy oder 3-7C-Cycloalkylmethoxy oder ganz oder teilweise durch Fluor substituiertes 1-2C-Alkoxy bedeutet,
R3 Wasserstoff bedeutet,
R31 Wasserstoff bedeutet,

oder worin

R3 und R31 gemeinsam eine 1-2C-Alkylengruppe bedeuten,
R4 Wasserstoff oder 1-4C-Alkyl bedeutet,
R5 Wasserstoff bedeutet,
R51 Wasserstoff bedeutet,

oder worin

R5 und R51 gemeinsam eine zusätzliche Bindung darstellen,
R6 einen durch R7 substituierten Phenylrest darstellt, wobei
R7 COOR71 oder CON(R72)R73 bedeutet und

R71 Wasserstoff, 1-7C-Alkyl oder 3-7C-Cycloalkylmethyl bedeutet und
R72 und R73 unabhängig voneinander Wasserstoff oder 1-7C-Alkyl bedeuten,

sowie die Salze dieser Verbindungen.

Besonders hervorzuhebende Verbindungen der Formel I sind solche, worin

R1 1-4C-Alkoxy, 3-7C-Cycloalkoxy oder ganz oder teilweise durch Fluor substituiertes 1-2C-Alkoxy bedeutet,
R2 1-4C-Alkoxy, 3-7C-Cycloalkoxy oder ganz oder teilweise durch Fluor substituiertes 1-2C-Alkoxy bedeutet,
R3 Wasserstoff bedeutet,

R31 Wasserstoff bedeutet,

oder worin

R3 und R31 gemeinsam eine 1-2C-Alkylengruppe bedeuten,
R4 Wasserstoff oder 1-4C-Alkyl bedeutet,
R5 Wasserstoff bedeutet,
R51 Wasserstoff bedeutet,

oder worin

R5 und R51 gemeinsam eine zusätzliche Bindung darstellen,
R6 einen durch R7 substituierten Phenylrest darstellt, wobei
R7 COOR71 oder CON(R72)R73 bedeutet und

R71 Wasserstoff, 1-7C-Alkyl oder 3-7C-Cycloalkylmethyl bedeutet und
R72 und R73 unabhängig voneinander Wasserstoff oder 1-4C-Alkyl bedeuten,

sowie die Salze dieser Verbindungen.

Bevorzugte Verbindungen der Formel I sind solche, in denen

R1 Methoxy, Ethoxy oder Difluormethoxy bedeutet,
R2 Methoxy, Ethoxy oder Difluormethoxy bedeutet,
R3 und R31 Wasserstoff bedeuten,
R4 Wasserstoff bedeutet,
R5 und R51 Wasserstoff bedeuten,
R6 einen durch R7 substituierien Phenylrest darstellt, wobei
R7 COOR71 oder CON(R72)R73 bedeutet und

R71 Wasserstoff oder 1 -4C-Alkyl bedeutet,
R72 Wasserstoff bedeutet und
R73 1-4C-Alkyl bedeutet,

sowie die Salze dieser Verbindungen.

Eine Ausgestaltung der bevorzugten Verbindungen der Formel I sind solche, in denen

R1 Methoxy oder Ethoxy bedeutet,
R2 Methoxy, Ethoxy oder Difluormethoxy bedeutet,
R3 und R31 Wasserstoff bedeuten,
R4 Wasserstoff bedeutet,
R5 und R51 Wasserstoff bedeuten,
R6 einen durch R7 substituierten Phenylrest darstellt, wobei
R7 COOR71 bedeutet und

R71 Wasserstoff oder 1-4C-Alkyl bedeutet,

sowie die Salze dieser Verbindungen.

Als Salze kommen für Verbindungen der Formel I - je nach Substitution - alle Säureadditionssalze oder alle Salze mit Basen in Betracht. Besonders erwähnt seien die pharmakologisch verträglichen Salze der in der Galenik üblicherweise verwendeten anorganischen und organischen Säuren und Basen. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bemsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen auch Salze mit Basen in Betracht. Als Beispiele für Salze mit Basen seien Alkali- (Lithium-, Natrium-, Kalium-) oder Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Pharmakotogisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt.

Bei den Verbindungen der Formel I handelt es sich um chirale Verbindungen mit Chiralitätszentren in den Positionen 4a und 10b und je nach Bedeutung der Substituenten R3, R31, R4, R5 und R51 weiteren Chiralitätszentren in den Positionen 1,2,3 und 4. Die Erfindung umfaßt daher alle denkbaren reinen Diastereomeren und reinen Enantiomeren als auch deren Gemische in jedem Mischungsverhältnis, einschließlich der Racemate. Bevorzugt sind die Verbindungen der Formel I, in denen die Wasserstoffatome in den Positionen 4a und 10b cis-ständig zueinander sind. Insbesondere bevorzugt sind dabei die reinen cis-Diastereomeren und die reinen cis-Enantiomeren als auch deren Gemische in jedem Mischungsverhältnis und einschließlich der Racemate. Bevorzugt genannt seien dabei die (-)-cis-Enantiomeren.

Die Enantiomeren können in an sich bekannter Weise (beispielsweise durch Herstellung und Trennung entsprechender diastereoisomerer Verbindungen) separiert werden. Vorzugsweise erfolgt eine Enantiomerentrennung auf Stufe der Ausgangsverbindungen der Formel III (siehe beigefügtes Formelblatt). Alternativ können enantiomerenreine Ausgangsverbindungen der Formel III auch über asymmetrische Synthesen dargestellt werden.

Weiterer Gegenstand der Erfindung ist ein Verfahren zur Herstellung der Verbindungen der Formel I, worin R1, R2, R3, R31, R4, R5, R51 und R6 die oben angegebenen Bedeutungen besitzen, und ihrer Salze. Das Verfahren ist dadurch gekennzeichnet, daß man Verbindungen der Formel II (siehe beigefügtes Formelblatt), in denen R1, R2, R3, R31, R4, R5, R51 und R6 die oben angegebenen Bedeutungen besitzen, cyclokondensiert und gewünschtenfalls anschließend erhaltene Verbindungen der Formel I in ihre Salze überführt, oder daß man gewünschtenfalls anschließend erhaltene Salze der Verbindungen der Formel I in die freien Verbindungen überführt.

Gewünschtenfalls können erhaltene Verbindungen der Formel I durch Derivatisierung in weitere Verbindungen der Formel I übergeführt werden. Beispielsweise können aus Verbindungen der Formel I, worin R7 eine Estergruppe darstellt durch saure oder alkalische Verseifung die entsprechenden Säuren erhalten werden, durch Umsetzung mit Aminen der Formel HN(R72)R73 die entsprechenden Amide dargestellt werden oder auch durch Umesterung von Estern der Formel I bzw. durch Veresterung von Säuren der Formel I entsprechende Ester hergestellt werden. Die Umsetzungen erfolgen zweckmäßigerweise analog dem Fachmann bekannter Methoden, z.B. so wie in den nachfolgenden Beispielen beschrieben.

Die Cyclokondensation erfolgt auf eine dem Fachmann an sich bekannte Weise gemäß Bischler-Napieralski (z.B. so, wie in J. Chem. Soc., **1956**, 4280-4282 beschrieben) in Gegenwart eines geeigneten Kondensationsmittels, wie beispielsweise Polyphosphorsäure, Phosphorpentachlorid, Phosphorpentoxid oder bevorzugt Phosphoroxytrichlorid, in einem geeigneten inerten Lösungsmittel, z.B. in einem chlorierten Kohlenwasserstoff wie Chloroform, oder in einem cyclischen Kohlenwasserstoff wie Toluol oder Xylol, oder einem sonstigen inerten Lösungsmittel wie Acetonitril, oder ohne weiteres Lösungsmittel unter Verwendung eines Überschusses an Kondensationsmittel, vorzugsweise bei erhöhter Temperatur, insbesondere bei der Siedetemperatur des verwendeten Lösungs- bzw. Kondensationsmittels.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel, z.B. in einem chlorierten Kohlenwasserstoff, wie Methylenchlorid oder Chloroform, oder einem niederrnolekularen aliphatischen Alkohol (Methanol, Ethanol, Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuem in die freien Verbindungen umgewandelt werden, welche wiederum in Salze übergeführt werden können. Auf diese Weise lassen sich pharmakologisch nicht verträgliche Salze in pharmakologisch verträgliche Salze umwandeln.

Verbindungen der Formel II (siehe beigefügtes Formelblatt), worin R1, R2, R3, R31, R4, R5, R51 und R6 die oben angegebenen Bedeutungen haben, sind aus den entsprechenden Verbindungen der Formel III (siehe beigefügtes Formelblatt), worin R1, R2, R3, R31, R4, R5 und R51 die oben angegebenen Bedeutungen haben, durch Umsetzung mit Verbindungen der Formel R6-CO-X, worin R6 die oben angegebene Bedeutung hat und X eine geeignete Abgangsgruppe, vorzugsweise ein Chloratom darstellt, zugänglich. Beispielsweise wird die Benzoylierung wie in den nachfolgenden Beispielen oder wie in J. Chem. Soc. (C), **1971**,1805-1808 beschrieben durchgeführt.

Verbindungen der Formel R6-CO-X und Verbindungen der Formel III sind entweder bekannt oder können auf bekannte Weise hergestellt werden.

Die Verbindungen der Formel III lassen sich z.B. aus Verbindungen der Formel IV (siehe beigefügtes Formelblatt), worin R1, R2, R3, R31, R4, R5 und R51 die oben genannten Bedeutungen haben, durch Reduktion der Nitrogruppe darstellen.

Die Reduktion erfolgt auf eine dem Fachmann bekannte Weise, beispielsweise so wie in J. Org. Chem. **1962**, 27, 4426 oder wie in den folgenden Beispielen beschrieben. Vorzugsweise erfolgt die Reduktion durch katalytische Hydrierung, z.B. in Gegenwart von Raney-Nickel, in einem niederen Alkohol wie Methanol oder Ethanol bei Raumtemperatur und unter Normal- oder erhöhtem Druck. Gewünschtenfalls kann dem Lösungsmittel eine katalytische Menge einer Säure, wie beispielsweise Salzsäure zugesetzt werden.

Die Gewinnung enantiomerenreiner Verbindungen der Formel III erfolgt auf eine dem Fachmann bekannte Weise z.B. über Salzbildung der racemischen Verbindungen der Formel III mit optisch aktiven Carbonsäuren.

Alternativ lassen sich enantiomerenreine Verbindungen der Formel III auch durch asymmetrische Synthese ausgehend von Verbindungen der Formel IVa (siehe beigefügtes Formelblatt) nach Iminbildung mit optisch aktiven Aminen (z.B. R-(+)-1-Phenethylamin und S-(-)-1-Phenethylamin) durch Hydrierung und anschließende reduktive Spaltung des erhaltenen sekundären Amins gewinnen (z.B. wie in Arch. Pharm. **1989**,322.187 oder wie in den folgenden Beispielen beschrieben).

Verbindungen der Formel IVa können beispielsweise ausgehend von Verbindungen der Formel IV in einer dem Fachmann bekannten Weise (z.B. so wie in Tetrahedron **1968**, 24, 6583 oder wie in den Beispielen beschrieben) erhalten werden.

Die Verbindungen der Formel IV (siehe beigefügtes Formelblatt), worin R1, R2, R3, R31 und R4 die oben angegebenen Bedeutungen haben und R5 und R51 Wasserstoff bedeuten, sind entweder bekannt oder können aus entsprechenden Verbindungen der Formel IV, worin R5 und R51 gemeinsam eine weitere Bindung bedeuten, hergestellt werden. Die Reaktion kann auf eine dem Fachmann bekannte Weise erfolgen, vorzugsweise durch Hydrierung in Gegenwart eines Katalysators, wie beispielsweise Palladium auf Aktivkohle, z.B. so wie in J. Chem. Soc.(C), **1971**, 1805-1808 oder wie in den folgenden Beispielen beschrieben.

Die Verbindungen der Formel IV, worin R5 und R51 gemeinsam eine weitere Bindung bedeuten, sind entweder bekannt oder können durch Umsetzung von Verbindungen der Formel V (siehe beigefügtes Formelblatt), worin R1 und R2 die oben genannten Bedeutungen haben, mit Verbindungen der Formel VI (siehe beigefügtes Formelblatt), worin R3, R31 und R4 die oben genannten Bedeutungen besitzen, erhalten werden.

Die Cycloaddition erfolgt dabei auf eine dem Fachmann bekannte Weise gemäß Diels-Alder, z.B. so wie in J. Amer. Chem. Soc. **1957**, 79, 6559 oder in J. Org. Chem. **1952**,17,581 oder wie in den folgenden Beispielen beschrieben.

Bei der Cycloaddition erhaltene Verbindungen der Formel IV, worin der Phenylring und die Nitrogruppe transständig zueinander sind, können in einer dem Fachmann bekannten Weise in die entsprechenden cis-Verbindungen übergeführt werden z.B. so wie in J. Amer. Chem. Soc. **1957**, 79, 6559 oder wie in den nachfolgenden Beispielen beschrieben.

Die Verbindungen der Formel VI und V sind entweder bekannt oder können auf bekannte Weise hergestellt werden. Die Verbindungen der Formel V können beispielsweise auf eine dem Fachmann bekannte Weise aus entsprechenden Verbindungen der Formel VII, so wie z.B. in J. Chem. Soc. **1951**, 2524 oder in J. Org. Chem. **1944**, 9, 170 oder wie in den folgenden Beispielen beschrieben, hergestellt werden.

Die Verbindungen der Formel VII (siehe beigefügtes Formelblatt), worin R1 und R2 die oben angegebenen Bedeutungen haben, sind entweder bekannt oder können auf eine dem Fachmann bekannte Weise, so wie z.B. in Ber. Dtsch. Chem. Ges. **1925**, 58, 203 oder wie in den folgenden Beispielen beschrieben, hergestellt werden.

Die nachfolgenden Beispiele dienen der näheren Erläuterung der Erfindung ohne sie einzuschränken. Ebenso können weitere Verbindungen der Formeln I, deren Herstellung nicht explizit beschrieben ist, in analoger oder in einer dem Fachmann an sich vertrauten Weise unter Anwendung üblicher Verfahrenstechniken hergestellt werden.

In den Beispielen steht Schmp. für Schmelzpunkt, d.Th. für der Theorie, Sdp. für Siedepunkt, h für Stunde (n), RT für Raumtemperatur, SF für Summenformel, MG für Molgewicht, Ber. für Berechnet, Gef. für Gefunden. Die in den Beispielen genannten Verbindungen und ihre Salze sind bevorzugter Gegenstand der Erfindung.

## Beispiele

### Endprodukte

1. **(+/-)-cis-8,9-Dimethoxy-6-[4-(methoxycarbonyl)phenyl]-1,2,3,4,4a,10b-hexahydrophenanthridin**

1,8g (+/-)-cis-N-[2-(3,4-Dimethoxyphenyl)cyclohexyl]-4-methoxycarbonylbenzamid werden in 50ml Acetonitril und 1,0 ml Phosphoroxychlorid gelöst und 8 h bei 50°C gerührt. Das Reaktionsgemisch wird in 100 ml gesättigte Natriumhydrogencarbonatlösung gegeben und mit Essigsäureethylester extrahiert. Die organische Phase wird mit

Natriumhydrogencarbonatlösung und Wasser gewaschen, mit Natriumsulfat getrocknet und eingeengt. Der Rückstand wird aus Essigsäureethylester/Petroläther umkristallisiert. Man erhält 660 mg (38,6 % d.Th.) der Titelverbindung mit Schmp.: 121,5-122,5°C.
SF: $C_{23}$ $H_{25}$ N $O_4$; MG: 379,46

| | | | | |
|---|---|---|---|---|
| Elementaranalyse | Ber. | C 72,80 | H 6,64 | N 3,69 |
| | Gef. | C 72,70 | H 6,61 | N 3,58 |

Ausgehend von den nachfolgend beschriebenen entsprechenden Ausgangsverbindungen erhält man analog der Arbeitsweise nach Beispiel 1:

2. **(+/-)-cis-8,9-Diethoxy-6-[4-(methoxycarbonyl)phenyl]-1,2,3,4a,10b-hexahydrophenanthridin**

Schmp.: 136-137°C, Ausbeute 59,8 % d.Th.
SF: $C_{25}$ $H_{29}$ N $O_4$; MG: 407,51

| | | | | |
|---|---|---|---|---|
| Elementeranalyse | Ber. | C 73,69 | H 7,18 | N 3,44 |
| | Gef. | C 73,83 | H 7,27 | N 3,59 |

3. **(+/-)-cis-9-Difluomethoxy-8-methoxy-6-[4-(methoxycarbonyl)phenyl]-1,2,3,4,4a,10b-hexahydrophenanthridin**

Schmp.: 126-127°C, Ausbeute 34,5 % d.Th.
SF: $C_{23}$ $H_{23}$ $F_2$ N $O_4$; MG: 415,44

| | | | | | |
|---|---|---|---|---|---|
| Elementaranalyse | Ber. | C 66,50 | H 5,58 | N 3,37 | F 9,15 |
| | Gef. | C 66,59 | H 5,55 | N 3,36 | F 9,21 |

4. **(+/-)-cis-9-Ethoxy-8-methoxy-6-[4-(methoxycarbonyl)phenyl]-1,2,3,4,4a,10b-hexahydrophenanthridin**

Schmp.: 143,5-144.5°C, Ausbeute: 88,6 % d.Th.
SF: $C_{24}$ $H_{27}$ N $O_4$; MG: 393,48

| | | | | |
|---|---|---|---|---|
| Elementaranalyse | Ber. | C 73,26 | H 6,92 | N 3,56 |
| | Gef. | C 73,24 | H 6,92 | N 3,70 |

5. **(+/-)-cis-8-Ethoxy-9-methoxy-6-[4-(methoxycarbonyl)phenyl]-1,2,3,4,4a,10b-hexahydrophenanthridin**

Schmp.: 123-124,5°C; Ausbeute: 81,9 % d.Th.
SF: $C_{24}$ $H_{27}$ N $O_4$ ; MG: 393,48

| | | | | |
|---|---|---|---|---|
| Elementaranalyse | Ber. | C 73,26 | H 6,92 | N 3,56 |
| | Gef. | C 73,37 | H 6,97 | N 3,56 |

6. **(+/-)-cis-8,9-Diethoxy-6-[3-(methoxycarbonyl)phenyl]-1,2,3,4,4a,10b-hexahydrophenanthridin**

Öl; Ausbeute: 40,4 % d.Th.
SF: $C_{23}$ $H_{29}$ N $O_4$; MG: 407,51

| | | | | |
|---|---|---|---|---|
| Elementaranalyse | Ber. | C 73,69 | H 7,17 | N 3,44 |
| | Gef. | C73,10 | H7,10 | N3,33 |

7. **(+/-)-cis-8,9-Diethoxy-6-[2-(methoxycarbonyl)phenyl]-1,2,3,4,4a,10b-hexahydrophenanthridin**

Öl; Ausbeute: 19,2 % d.Th.

SF: $C_{25} H_{29} N O_4$ ; MG: 407,51

| | | | | |
|---|---|---|---|---|
| Elementaranalyse x 0,5 $H_2O$ | Ber. | C 72,79 | H 7,26 | N 3,36 |
| | Gef. | C 71,90 | H 7,26 | N 3,20 |

8. **(+/-)-trans-8,9-Dimethoxy-6-[4-(methoxycarbonyl)phenyl]-1,2,3,4,4a,10b-hexahydrophenanthridin**

Schmp.: 212-214°C; Ausbeute: 52,7 % d.Th.
SF: $C_{23} H_{25} N O_4$; MG: 379,46

| | | | | |
|---|---|---|---|---|
| Elementaranalyse | Ber. | C 72,80 | H 6,64 | N 3,69 |
| | Gef. | C 72,69 | H 6,68 | N 3,67 |

9. **(-)-cis-8,9-Diethoxy-6-[4-(methoxycarbonyl)phenyl]-1,2,3,4,4a,10b-hexahydrophenanthridin**

Schmp.: 92-95°C; Ausbeute: 46,6 % d.Th.
SF: $C_{25} H_{29} N O_4$; MG: 407,51
Drehwert: $[\alpha]_D^{20}$ -61.26° (c = 0,475, Ethanol)

| | | | | |
|---|---|---|---|---|
| Elementaranalyse: | Ber.: | C 73,69 | H 7,17 | N 3,44 |
| | Gef.: | C 73,55 | H 7,25 | N 3,35 |

10. **(+)-cis-8,9-Diethoxy-6-[4-(methoxycarbonyl)phenyl]-1,2,3,4,4a,10b-hexahydrophenanthridin**

Schmp.: 92-125°C; Ausbeute: 48,2 % d.Th.
SF: $C_{25} H_{29} N O_4$; MG: 407,51
Drehwert: $[\alpha]_D^{20}$ +60.08° (c = 0,23, Ethanol)

| | | | | |
|---|---|---|---|---|
| Elementaranalyse | Ber. | C73;69 | H 7,17 | N 3,44 |
| | Gef. | C 73,66 | H 7,20 | N 3,60 |

11. **(-)-cis-8,9-Dimethoxy-6-[4-(methoxycarbonyl)phenyl]-1,2,3,4,4a,10b-hexahydrophenanthridin**

Erstarrendes Öl; Ausbeute: 58,1 % d.Th.
SF: $C_{23} H_{25} N O_4$ ; MG: 379,46
Drehwert: $[\alpha]_D^{20}$ -90,0° (c = 0,2, Ethanol)

| | | | | |
|---|---|---|---|---|
| Elementaranalyse | Ber. | C 72,80 | H 6,64 | N 3,69 |
| | Gef. | C 72,80 | H 6,90 | N 3,54 |

12. **(+)-cis-8,9-Dimethoxy-6-[4-(methoxycarbonyl)phenyl]-1,2,3,4,4a,10b-hexahydrophenanthridin**

Erstarrendes Öl; Ausbeute: 86,9 % d.Th.
SF: $C_{23} H_{25} N O_4$; MG: 379,46
Drehwert: $[\alpha]_D^{20}$ +83,9° (c = 0,2, Ethanol)

| | | | | |
|---|---|---|---|---|
| Elementaranalyse | Ber.: | C 72,80 | H 6,64 | N 3,69 |
| | Gef. | C 72,99 | H 6,73 | N 3,66 |

13. **(+/-)-cis-8,9-Dimethoxy-6-[4-cyclopropylmethoxycarbonyl)phenyl]-1,2,3,4,4a,10b-hexahydrophenanthridin**

2,0 g (+/-)-cis-8,9-Dimethoxy-6-(4-carboxyphenyl)-1,2,3,4,4a,10b-hexahydrophenanthridin werden in 12,0 ml Cyclopropylmethanol suspendiert, mit 1,0 ml Thionylchlorid versetzt und 4 Tage bei 50°C gerührt. Die Lösung wird unter vermindertem Druck eingeengt und der Rückstand zwischen Essigsäureethylester und verdünnter Natriumhydrogencarbonat-Lösung verteilt. Die organische Phase wird mit Natriumsulfat getrocknet und eingeengt. Der Rückstand wird aus Essigsäureethylester umkristallisiert.
Man erhält 1,33 g (63.7 % d.Th.) der Titelverbindung mit Schmp.: 147-148°C.
SF: $C_{26}$ $H_{29}$ N $O_4$; MG: 419,53

| | | | | |
|---|---|---|---|---|
| Elementaranalyse | Ber. | C 74,44 | H 6,97 | N 3,34 |
| | Gef. | C 74,13 | H 6,84 | N 3,48 |

14. **(+/-)-cis-6-(4-Carboxyphenyl)-8,9-diethoxy-1,2,3,4,4a,10b-hexahydro-phenanthridin-hydrochlorid**

220 mg (+/-)-cis-8,9-Diethoxy-6-[4-(methoxycarbonyl)phenyl]-1,2,3,4,4a,10b-hexahydrophenanthridin werden in 3,0 ml konz. Salzsäure und 5,0 ml Wasser gelöst und 4 h bei 80°C gerührt. Der beim Abkühlen entstehende Niederschlag wird abgesaugt und getrocknet. Man erhält 135 mg (58,1 % d.Th.) der Titelverbindung mit Schmp.: 269°C.
SF: $C_{24}$ $H_{27}$ N $O_4$ x HCl; MG: 429,95

| | | | | | |
|---|---|---|---|---|---|
| Elementaranalyse | Ber. | C 67,05 | H 6,56 | Cl 8,25 | N 3,26 |
| | Gef. | C 66,90 | H 6,51 | Cl 8,22 | N 3,11 |

Ausgehend von den vorstehend beschriebenen entsprechenden Ausgangsverbindungen erhält man analog der Arbeitsweise nach Beispiel 14:

15. **(+/-)-cis-6-(4-Carboxyphenyl)-8,9-dimethoxy-1,2,3,4,4a,10b-hexahydro-phenanthridin-hydrochlorid**

Schmp. 220°C (Zers.); Ausbeute 65 % d.Th.
SF: $C_{22}$ $H_{23}$ N $O_4$ x HCl; MG: 401,89

| | | | | | |
|---|---|---|---|---|---|
| Elementaranalyse | Ber. | C 65,75 | H 6,02 | Cl 8,82 | N 3,48 |
| | Gef. | C 65,63 | H 6,06 | Cl 8,58 | N 3,60 |

16. **(+/-)-cis-6-(4-Carboxyphenyl)-9-ethoxy-8-methoxy-1,2,3,4,4a,10b-hexahydrophenanthridin-hydrochlorid**

Schmp.: 180°C (Zers.); Ausbeute: 53,4 % d.Th.
SF: $C_{23}$ $H_{25}$ N $O_4$ x HCl; MG: 415,92

| | | | | | |
|---|---|---|---|---|---|
| Elementaranalyse x 2,5 $H_2O$ | Ber. | C 59,93 | H 6,78 | N 3,03 | Cl 7,68 |
| | Gef. | C 59.70 | H 6,57 | N 3,18 | Cl 7,71 |

17. **(+/-)-cis-6-(4-Carboxyphenyl)-8-ethoxy-9-methoxy-1,2,3,4,4a,10b-hexahydrophenanthridin-hydrochlorid**

Schmp.: > 250°C, Ausbeute: 32,4 % d.Th.
SF: $C_{23}$ $H_{25}$ N $O_4$ x HCl, MG: 415,92

| | | | | | |
|---|---|---|---|---|---|
| Elementaranalyse | Ber. | C 66,42 | H 6,30 | N 3,37 | Cl 8,52 |
| | Gef. | C 66,37 | H 6,31 | N 3,24 | Cl 8,79 |

18. **(+/-)-cis-6-(3-Carboxyphenyl)-8,9-diethoxy-1,2,3,4,4a,10b-hexahydrophenanthridin-hydrochlorid**

Schmp.: 259-260°C, Ausbeute: 32,6 % d.Th.
SF: $C_{24}$ $H_{27}$ N $O_4$ x HCl; MG: 429,95

| | | | | | |
|---|---|---|---|---|---|
| Elementaranalyse | Ber. | 67,05 | H 6,56 | N 3,26 | Cl 8,25 |
| | Gef. | C 67,05 | H 6,67 | N 3,19 | Cl 8,22 |

19. **(+/-)-cis-6-(4-Carboxyphenyl)-9-difluormethoxy-8-methoxy-1,2,3,4,4a,10b-hexahydrophenanthridin-hydrochlorid**

Schmp.: 170°C (Zers.), Ausbeute: 69,8 % d.Th.
SF: $C_{22}$ $H_{21}$ $F_2$ N $O_4$ x HCl, MG: 437,88

| | | | | | | |
|---|---|---|---|---|---|---|
| Elementaranalyse x $C_2H_5OH$ | Ber. | C 59,57 | H 5,83 | N 2,89 | F 7,85 | Cl 7,33 |
| | Gef. | C 59,39 | H 5,76 | N 2,65 | F 8,00 | Cl 7,10 |

20. **(+/-)-cis-6-[4-(N-methylaminocarbonyl)phenyl]-8,9-dimethoxy-1,2,3,4,4a,10b-hexahydrophenanthridin**

680 mg (+/-)-cis-8,9-Dimethoxy-6-[4-(methoxycarbonyl)phenyl]-1,2,3,4,4a,10b-hexahydrophenanthridin werden mit 25 mg Natriumcyanid in 13 ml 9 molarer Methylamin-Lösung in Ethanol im Autoklaven 10 h auf 90°C erwärmt. Die Lösung wird im Vakuum eingeengt, der Rückstand in Methylenchlorid aufgenommen und mit Wasser extrahiert. Die organische Phase wird mit Natriumsulfat getrocknet, das Lösungsmittel im Vakuum entfernt, der Rückstand in Diethylether ausgerührt, abgesaugt und getrocknet. Man erhält 380 mg (56,1 % d.Th.) der Titelverbindung mit Schmp. 209-211°C.
SF: $C_{23}$ $H_{26}$ $N_2$ $O_3$: MG: 378,48

| | | | | |
|---|---|---|---|---|
| Elementaranalyse | Ber. | C 72,99 | H 6,92 | N 7,40 |
| | Gef. | C 72,64 | H 6,99 | N 7,44 |

**Ausgangsverbindungen**

A1. **(+/-)-cis-N-[2-(3,4-Dimethoxyphenyl)cyclohexyl]-4-methoxycarbonylbenzamid**

2,6 g (+/-)-cis-1,2-Dimethoxy-4-(2-aminocyclohexyl)benzol werden in 20 ml Methylenchlorid und 1 ml Triethylamin gelöst. Man tropft bei RT innerhalb von 3 h eine Lösung von 2,4 g 4-Methoxycarbonylbenzoesäurechlorid in 30 ml Methylenchlarid zu, extrahiert nach 1 h Rühren mit je 50 ml Wasser, 2N Salzsäure, ges. Natriumhydrogencarbonatlösung und nochmals Wasser. Die organische Phase wird mit Natriumsulfat getrocknet, eingeengt und aus Essigsäureethylester kristallisiert. Man erhält 2,0 g (45,5 % d.Th.) der Titelverbindung mit Schmp. 139-143°C.
Ausgehend von den nachfolgend beschriebenen entsprechenden Ausgangsverbindungen erhält man analog der Arbeitsweise nach Beispiel A1:

A2. **(+/-)-cis-N-[2-(3-Difluormethoxy-4-methoxyphenyl)cyclohexyl]-4-methoxy-carbonylbenzamid**

Öl; Ausbeute 96,5 % d.Th.

A3. **(+/-)-cis-N-[2-(3,4-Diethoxyphenyl)cyclohexyl]-4-methoxycarbonylbenzamid**

Öl; Ausbeute 58,7 % d.Th.

A4. **(+/-)-cis-N-[2-(3-Ethoxy-4-methoxyphenyl)cyclohexyl]-4-methoxycarbonylbenzamid**

Schmp.: 151,5-152.5°C; Ausbeute: 78,9 % d.Th.

A5. **(+/-)-cis-N-[2-(2-Ethoxy-3-methoxyphenyl)cyclohexyl]-4-methoxycarbonylbenzamid**

Schmp.: 126.5-127,5°C, Ausbeute: 58,7 % d.Th.

A6. **(+/-)-cis-N-[2-(2,3-Diethoxyphenyl)cyclohexyl]-3-methoxycarbonylbenzamid**

Öl; Ausbeute: 96,27 % d.Th.

A7. **(+/-)-cis-N-[2-(2,3-Diethoxyphenyl)cyclohexyl]-2-methoxycarbonylbenzamid**

Schmp.: 144-144,5°C; Ausbeute: 59,6 % d.Th.

A8. (**+/-)-trans-N[2-(3,4-Dimethoxyphenyl)cyclohexyl]-4-methoxycarbonylbenzamid**

Schmp.: 189-193-C, Ausbeute: 48,0 % d.Th.

A9. **(-)-cis-N-[2-(3,4-Diethoxyphenyl)cyclohexyl]-4-methoxycarbonylbenzamid**

Schmp.: 122-124°C. Ausbeute: 80,15 % d.Th.
Drehwert: $[\alpha]_D^{20}$ -137,3° (c = 0,11, Ethanol)

A10. (**+)-cis-N-[2-(3,4-Diethoxyphenyl)cyclohexyl]-4-methoxycarbonylbenzamid**

Schmp.: 123-125°C, Ausbeute: 86,75 % d.Th.
Drehwert: $[\alpha]_D^{20}$ +134,8° (c = 0,135, Ethanol)

A11. **(-)-cis-N-[2-(3,4-Dimethoxyphenyl)cyclohexyl]-4-methoxycarbonylbenzamid**

Schmp.: 154,5-156°C, Ausbeute: 85,2 % d.Th.
Drehwert: $[\alpha]_D^{20}$ -167,7° (c = 0,2, Ethanol)

**A12. (+)-cis-N-[2-(3,4-Dimethoxyphenyl)cyclohexyl]-4-methoxycarbonylbenzamid**

Schmp.: 153,5-154,5°C, Ausbeute: 85,1 % d.Th.
Drehwert: $[\alpha]_D^{20}$ +165° (c = 0,2, Ethanol)

B1. **(+/-)-cis-1,2-Dimethoxy-4-(2-aminocyclohexyl)benzol**

8,5 g (+/)-cis-1,2-Dimethoxy-4-(2-nitrocyclohexyl)benzol werden in 400 ml Methanol gelöst und bei RT innerhalb von 8 h portionsweise mit 7 ml Hydrazinhydrat und 2,5 g Raney-Nickel versetzt. Nach Rühren über Nacht bei RT wird das Reaktionsgemisch filtriert, das Filtrat eingeengt und der Rückstand über Kieselgel mit einer Mischung aus Toluol/Essigsäureethylester/Triethylamin = 4/2/0,5 chromatographiert.
Öl; Ausbeute 74,4 % d.Th.

Ausgehend von den nachfolgend beschriebenen Ausgangsverbindungen erhält man entspechend der Arbeitsweise nach Beispiel B1:

B2. **(+/-)-trans-1,2-Dimethoxy-4-(2-aminocyclohexyl)benzol**

Öl; Ausbeute: 65,9 % d.Th.

B3. **(+/-)-cis-1,2-Diethoxy-4-(2-aminocyclohexyl)benzol**

Öl; Ausbeute 42,8 % d.Th.

B4. **(+/-)-cis-2-Difluormethoxy-1-methoxy-4-(2-aminocyclohexyl)benzol**

14,6 g (+/-)-cis-2-Difluormethoxy-1-methoxy-4-(2-nitrocyclohex-4-enyl)benzol werden in 170 ml Ethanol gelöst, mit 3,0 g Raney-Nickel versetzt und bei 50 bar Wasserstoffdruck 9 Tage im Autoklaven hydriert. Die Suspension wird filtriert, das Lösungsmittel im Vakuum entfernt und der Rückstand über Kieselgel mit einer Mischung aus Toluol/Dioxan/Triethylamin 3/1/0,5 chromatographiert. Nach Eindampfen entsprechender Fraktionen erhält man 3,4 g (25,7 % d.Th.) der Titelverbindung als Öl.

Ausgehend von den nachfolgend beschriebenen entsprechenden Ausgangsverbindungen erhält man analog der Arbeitsweise nach Beispiel B4:

B5. **(+/-)-cis-2-Ethoxy-1-methoxy-4-(2-aminocyclohexy)benzol**

Öl; Ausbeute quantitativ

B6. **(+/-)-cis-1-Ethoxy-2-methoxy-4-(2-aminocyclohexyl)benzol**

Öl; Ausbeute quantitativ

B7. **(-)-cis-1,2-Diethoxy-4-(2-aminocyclohexyl)benzol-Hydrochlorid**

2,2 g (-)-cis-1,2-Diethoxy-4-[2-(1-phenylethyl)aminocyclohexyl]benzol werden in 50 ml Ethanol suspendiert, mit 270 mg 10%iger Palladiumkohle versetzt und bei 50°C und 50 bar Wasserstoffdruck 6 Tage hydriert. Nach Abfiltrieren des Katalysators wird die Lösung unter vermindertem Druck aufkonzentriert und das kristallisierende Produkt abgesaugt und getrocknet.
Schmp.: 145-147°C ; Ausbeute: 59,0 % d.Th.
Drehwert: $[\alpha]_D^{20}$ -60° (c = 0,12, Ethanol)
Ausgehend von den nachfolgend beschriebenen entsprechenden Ausgangsverbindungen erhält man analog der Arbeitsweise nach Beispiel B7:

B8. **(+)-cis-1,2-Diethoxy-4-(2-aminocyclohexyl)benzol-Hydrochlorid**

Schmp.: 149-151°C : Ausbeute: 52,9 % d.Th.
Drehwert: $[\alpha]_D^{20}$ +70° (c = 0,21, Ethanol)

B9. **(-)-cis-1,2-Dimethoxy-4-(2-aminocyclohexyl)benzol**

12,0 g (+/-)-cis-1,2-Dimethoxy-4-(2-aminocyclohexyl)benzol und 6,2 g (-)-Mandelsäure werden in 420 ml Dioxan und 60 ml Tetrahydrofuran gelöst und über Nacht bei RT gerührt. Der Feststoff wird abgesaugt, getrocknet, mit 100 ml gesättigter Natriumhydrogencarbonatlösung versetzt und mit Essigsäureethylester extrahiert. Die organische Phase wird mit Natriumsulfat getrocknet und unter vermindertem Druck eingeengt. Man erhält 4,8 g (40,0 % d. Th.) der Titelverbindung mit Schmp.: 80-81,5°C.
Drehwert: $[\alpha]_D^{20}$ -58,5° (c = 1, Ethanol).
Ausgehend von den nachfolgend beschriebenen entsprechenden Ausgangsverbindungen erhält man analog der Arbeitsweise nach Beispiel B9:

B10. **(+)-cis-1,2-Dimethoxy-4-(2-aminocyclohexyl)benzol**

Schmp.: 68-69°C; Ausbeute 37,2 % d.Th.
Drehwert: $[\alpha]_D^{20}$ +59.2° (c = 1, Ethanol)

C1. **(-)-cis-1,2-Diethoxy-4-[2-(1-phenylethyl)aminocyclohexyl]benzol**

4,0 g 2-(3,4-Diethoxyphenyl)cyclohexanon, 2,0 ml R-(+)-1-Phenethylamin und 20 mg Toluolsulfonsäure werden in 150 ml Toluol gelöst und am Wasserabscheider 18 h unter Rückfluß erwärmt. Die Lösung wird unter vermindertem Druck eingeengt, der Rückstand in 200 ml Ethanol aufgenommen, mit 5 g ethanolfeuchtem Raney Nickel versetzt und bei RT und 30-60 bar Wasserstoffdruck 10 Tage hydriert. Man saugt vom Katalysator ab, engt die Lösung unter vermindertem Druck ein und chromatographiert den Rückstand über Kieselgel mit einem Gemisch aus Toluol/Dioxan/Triethylamin im Verhältnis 20/2/1. Nach Einengen der entsprechenden Eluatfraktionen erhält man 2,15 g (38,4 % d.Th.) der Titelverbindung als Öl.
Drehwert: $[\alpha]_D^{20}$ -3,7° (c = 0,27, Ethanol)
Ausgehend von den nachfolgend beschriebenen entsprechenden Ausgangsverbindungen erhält man analog der Arbeitsweise nach Beispiel C1:

C2. (**+)-cis-1,2-Diethoxy-4-[2-(1-phenylethyl)aminocyclohexyl]benzol**

Ausgehend von 2-(3,4-Diethoxyphenyl)cyclohexanon und S-(-)-1-Phenethylamin erhält man die Titelverbindung als Öl.
Ausbeute: 64,6 % d.Th.
Drehwert: $[\alpha]_D^{20}$ +7,1° (c = 0,56, Ethanol)

D1. **2-(3,4-Diethoxyphenyl)cyclohexanon**

10,2 g 2-(3,4-Diethoxyphenyl)cyclohex-4-enon in 600 ml Tetrahydrofuran werden mit 1,5 ml konz. Salzsäure und 400 mg 10%iger Palladiumkohle versetzt und hydriert. Nach Abfiltrieren des Katalysators und Einengen der Lösung unter vermindertem Druck wird der Rückstand über Kieselgel mit einem Gemisch aus Petrolether/Essigsäureethylester im Verhältnis 2/1 chromatographiert. Nach Einengen der entsprechenden Eluatfraktionen erhält man 8,25 g (80,9 % d.Th.) der Titelverbindung als erstarrendes Öl.

E1. **2-(3,4-Diethoxyphenyl)cyclohex-4-enon**

15,0 g (+/-)-trans-1,2-Diethoxy-4-(2-nitrocyclohex-4-enyl)benzol werden in 375 ml Ethanol gelöst und in 42 ml 20%ige Natriumethylatlösung getropft. Nach 20 min Rühren bei RT wird in eine eisgekühlte Lösung aus 67,5 ml konz. Salzsäure und 1,35 g Harnstoff in 225 ml Wasser und 170 ml Ethanol getropft. Die Lösung wird mit Wasser/Dichlormethan extrahiert, die organische Phase mit Natriumsulfat getrocknet und unter vermindertem Druck eingeengt. Der Rückstand wird über Kieselgel mit Petrolether/Essigsäureethylester im Verhältnis 2/1 chromatographiert. Nach Einengen der entsprechenden Eluatfraktionen erhält man 10,33 g (77,1 % d.Th.) der Titelverbindung als Öl.

F1. **(+/-)-cis-1,2-Dimethoxy-4-(2-nitrocyclohexyl)benzol**

8,4 g (+/-)-cis-1,2-Dimethoxy-4-(2-nitrocyclohex-4-enyl)benzol werden in 450 ml Methanol gelöst, mit 2 ml konz. Salzsäure versetzt und nach Zugabe von 500 mg Pd/C 10 %ig hydriert. Das Reaktionsgemisch wird filtriert und das Filtrat eingeengt. Schmp.: 84-86,5°C; Ausbeute quantitativ.

Ausgehend von den nachfolgend beschriebenen entsprechenden Ausgangsverbindungen erhält man analog der Arbeitsweise nach Beispiel F1:

F2. **(+/-)-cis-1,2-Diethoxy-4-(2-nitrocyclohexyl)benzol**

Öl; Ausbeute 96,5 % d.Th.

F3. **(+/-)-trans-1,2-Dimethoxy-4-(2-nitrocyclohexyl)benzol**

Öl; Ausbeute: 47,0 % d.Th.

G1. **(+/-)-cis-1,2-Dimethoxy-4-(-nitrocyclohex-4-enyl)benzol**

10,0 g (+/-)-trans-1,2-Dimethoxy-4-(2-nitrocyclohex-4-enyl)benzol und 20,0 Kaliumhydroxid werden in 150 ml Ethanol und 35 ml Dimethylformamid gelöst. Anschließend wird eine Lösung von 17,5 ml konz. Schwefelsäure in 60 ml Ethanol so zugetropft, daß die Innentemperatur 4°C nicht übersteigt. Nach 1 h Rühren wird auf 1 l Eiswasser gegeben, der Niederschlag abgesaugt, mit Wasser gewaschen, getrocknet und das Rohprodukt aus Ethanol umkristallisiert. Schmp. 82,5-84°C; Ausbeute 86 % d.Th.

Ausgehend von den nachfolgend beschriebenen entsprechenden Ausgangsverbindungen erhält man analog der Arbeitsweise nach Beispiel G1:

G2. **(+/-)-cis-2-Difluormethoxy-1-methoxy-4-(2-nitrocyclohex-4-enyl)benzol**

Öl; Ausbeute quantitativ

G3. **(+/-)-cis-1,2-Diethoxy-4-(2-nitrocyclohex-4-enyl)benzol**

Öl; Ausbeute 96,5 % d.Th.

G4. **(+/-)-cis-2-Ethoxy-1-methoxy-4-(2-nitrocyclohex-4-enyl)benzol**

Schmp.: 66-67°C; Ausbeute: 97,2 %

G5. **(+/-)-cis-1-Ethoxy-2-methoxy-4-(2-nitrocyclohex-4-enyl)benzol**

Schmp.: 96-97°C; Ausbeute 95,8 % d.Th.

H1. **(+/-)-trans-1,2-Dimethoxy-4-(2-nitrocyclohex-4-enyl)benzol**

50,0 3,4-Dimethoxy-ω-nitrostyrol und 1.0 g (9,1 mmol) Hydrochinon werden in 200 ml abs. Toluol suspendiert und bei -70°C mit 55,0 g (1,02 mol) flüssigem 1,3-Butadien versetzt. Die Mischung wird im Autoklaven 6 Tage bei 160°C gerührt und dann abgekühlt. Ein Teil des Lösungsmittels wird am Rotationsverdampfer entfernt, der entstehende Niederschlag wird abgesaugt und in Ethanol umkristallisiert. Schmp.: 113,5-115.5°C; Ausbeute 76,3 % d.Th.

Ausgehend von den nachfolgend beschriebenen entsprechenden Ausgangsverbindungen erhält man analog der Arbeitsweise nach Beispiel H1:

H2. **(+/-)-trans-2-Difluormethoxy-1-methoxy-4-(2-nitrocyclohex-4-enyl)benzol**

Schmp.: 100-102°C; Ausbeute 62,3 % d.Th.

H3. **(+/-)-trans-1,2-Diethoxy-4-(2-nitrocyclohex-4-enyl)benzol**

Schmp.: 80-81,5°C; Ausbeute 59,8 % d.Th.

H4. **(+/-)-trans-2-Ethoxy-1-methoxy-4-(2-nitrocyclohex-4-enyl)benzol**

Schmp.: 129.130°C: Ausbeute: 75,7 % d.Th.

H5. **(+/-)-trans-1-Ethoxy-2-methoxy-4-(2-nitrocyclohex-4-enyl)benzol**

Schmp.: 70,5-72°C; Ausbeute: 66,8 % d.Th.

I1. **3,4-Dimethoxy-ω-nitrostyrol**

207,0 g 3,4-Dimethoxybenzaldehyd, 100,0g Ammoniumacetat und 125ml Nitromethan werden in 1,0 l Eisessig 3-4 h zum Sieden erhitzt. Nach Abkühlen im Eisbad wird der Niederschlag abgesaugt, mit Eisessig und Petroläther nachgespült und getrocknet. Schmp.: 140-141°C. Ausbeute: 179,0g (68,5 % d.Th.).

Ausgehend von den nachfolgend beschriebenen oder literaturbekannten Ausgangsverbindungen erhält man analog der Arbeitsweise nach Beispiel I1:

I2. **3-Difluormethoxy-4-methoxy-ω-nitrostyrol**

Schmp.: 120-123°C; Ausbeute 24,8 % d.Th.

I3. **3,4-Diethoxy-ω-nitrostyrol**

Schmp.: 136-136,5°C; Ausbeute: 78,2% d.Th.

I4. **3-Ethoxy-4-methoxy-ω-nitrostyrol**

Schmp.: 132-133°C; Ausbeute: 70,3 % d.Th.

I5. **4-Ethoxy-3-methoxy-ω-nitrostyrol**

Schmp.: 147-148,5°C; Ausbeute: 68,6 % d.Th.

J. **3-Difluormethoxy-4-methoxybenzaldehyd**

In eine Mischung von 200 g Isovanillin, 6,7 g Benzyltrimethylammonium-chlorid, 314 g 50-proz. Natronlauge und 2 l Dioxan wird unter starkem Rühren ca. 2 h Chlordifluormethan eingeleitet. Anschließend wird die Mischung zwischen Eiswasser und Ethylacetat verteilt, die organische Phase abgetrennt, die wäßrige Phase 2 mal mit Ethylacetat ausgerührt, die vereinigten organischen Phasen über Magnesiumsulfat getrocknet und im Vakuum eingeengt. Zur Entfernung von nicht umgesetztem Isovanillin wird das Öl an neutralem Kieselgel mit Toluol chromatographiert. Nach dem Eindampfen des Eluats erhält man 249 g 3-Difluormethoxy-4-methoxybenzaldehyd als Öl.

## Gewerbliche Anwendbarkeit

Die erfindungsgemäßen Verbindungen besitzen wertvolle pharmakologische Eigenschaften, die sie gewerblich verwertbar machen. Als selektive Zyklisch-Nukleolid Phosphodiesterase (PDE) Inhibitoren (und zwar des Typs IV) eignen sie sich einerseits als Bronchialtherapeutika (zur Behandlung von Atemwegsobstruktionen aufgrund ihrer dilatierenden aber auch aufgrund ihrer atemfrequenz- bzw. atemantriebssteigemden Wirkung) und zur Behebung von erektiler Dysfunktion aufgrund der gefäßdilatierenden Wirkung, andererseits jedoch vor allem zur Behandlung von Erkrankungen, insbesondere entzündlicher Natur, z.B. der Atemwege (Asthma-Prophylaxe), der Haut, des Darms, der Augen, des zentralen Nervensystems und der Gelenke, die vermittelt werden durch Mediatoren, wie Histamin, PAF (Plättchen- aktivierender Faktor), Arachidonsäure-Abkömmlinge wie Leukotriene und Prostaglandine, Zytokine, Interleukine, Chemokine, alpha-, beta- und gamma-Interferon, Tumomekrosisfaktor (TNF) oder Sauerstoff-Radikale und Proteasen. Hierbei zeichnen sich die erfindungsgemäßen Verbindungen durch eine geringe Toxizität, eine gute enterale Resorption (hohe Bioverfügbarkeit), eine große therapeutische Breite und das Fehlen wesentlicher Nebenwirkungen aus.

Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophylaxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiale); Dermatosen (vor allem proliferativer, entzündlicher und allergischer Art) wie beispielsweise Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis der Arthritis (Rheumatoide Arthritis, Rheumatoide Spondylitis, Osteoarthritis und andere arthritische Zustände), Erkrankungen des Immunsystems (AIDS, Multiple Sklerose), Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; aber auch Erkrankungen des Herzens, die durch PDE-Hemmstoffe behandelt werden können, wie beispielsweise Herzinsuffizienz, oder Erkrankungen, die aufgrund der gewebsrelaxierenden Wirkung der PDE-Hemmstoffe behandelt werden können, wie beispielsweise erektile Dysfunktion oder Koliken der Nieren und der Hamleiter im Zusammenhang mit Nierensteinen.

Ein weiterer Gegenstand der Erfindung ist ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe der genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,01 und 1 mg pro Sprühstoß. Die übliche Dosis bei systemischer Therapie p.o. oder i.v. liegt zwischen 0,1 und 200 mg pro Applikation.

## Biologische Untersuchungen

Bei der Untersuchung der PDE IV-Hemmung auf zellulärer Ebene kommt der Aktivierung von Entzündungszellen besondere Bedeutung zu. Als Beispiel sei die FMLP (N-formyl-methionylleucyl-phenylalanin)-induzierte Superoxid-Produktion von neutrophilen Granulozyten genannt, die als Luminol-verstärkte Chemolumineszenz gemessen werden kann. [Mc Phail LC, Strum SL, Leone PA und Sozzani S, The neutrophil respiratory burst mechanism. In "Immunology Series" **1992**, 57, 47-76; ed. Coffey RG (Marcel Decker, Inc., New York-Basel-Hong Kong)].

Substanzen, welche die Chemolumineszenz sowie die Zytokinsekretion und die Sekretion entzündungssteigemder Mediatoren an Entzündungszellen, insbesondere die neutrophilen und eosinophilen Granulozyten. die T-Lymphozyten, die Monozyten und die Macrophagen hemmen, sind solche, welche die PDE IV hemmen. Dieses Isoenzym der Phosphodiesterase-Familien ist besonders in Granulozyten vertreten. Dessen Hemmung führt zur Erhöhung der intrazellulären zyklischen AMP-Konzentration und damit zur Hemmung der zellulären Aktivierung. Die PDE IV-Hemmung durch die erfindungsgemäßen Substanzen ist damit ein zentraler Indikator für die Unterdrückung von entzündlichen Prozessen. (**Giembycz MA**, Could isoenzyme-selective phosphodiesterase inhibitors render bronchodilatory therapy redundant in the treatment of bronchial asthma?. Biochem Pharmacol **1992**, 43, 2041-2051; **Torphy TJ** et al., Phosphodiesterase inhibitors: new opportunities for treatment of asthma. Thorax **1991**, 46, 512-523; **Schudt C** et al., Zardaverine: a cyclic AMP PDE III/IV inhibitor. In "New Drugs for Asthma Therapy", 379-402, Birkhäuser Verlag Basel 1991; **Schudt C** et al., Influence of selective phosphodiesterase inhibitors on human neutrophil functions and levels of cAMP and $Ca_i$. Naunyn-Schmiedebergs Arch Pharmacol **1991**, 344, 682-690; **Nielson CP** et al., Effects of selective phosphodiesterase inhibitors on polymorphonuclear leukocyte respiratory burst. J Allergy Clin Immunol **1990**, 86, 801-808; **Schade** et al., The specific type III and IV phosphodiesterase inhibitor zardaverine suppress formation of tumor necrosis factor by macrophages. European Journal of Pharmacology **1993**, 230, 9-14).

## Hemmung der PDE IV-Aktivität

### Methodik

Der Aktivitätstest wurde nach der Methode von Bauer und Schwabe durchgeführt, die auf Mikrotiterplatten adaptiert wurde (Naunyn-Schmiedeberg's Arch. Pharmacol. **1980**, 311, 193-198). Hierbei erfolgt im ersten Schritt die PDE-Reaktion. In einem zweiten Schritt wird das entstandene 5'-Nukleotid durch eine 5'-Nukleotidase des Schlangengiftes von Crotalus Atrox zum ungeladenen Nukleosid gespalten. Im dritten Schritt wird das Nukleosid auf Ionenaustauschsäulen vom verbliebenen geladenen Substrat getrennt. Die Säulen werden mit 2 ml 30 mM Ammoniumformiat (pH 6,0) direkt in Minivials eluiert, in die noch 2 ml Szintillatorflüssigkeit zur Zählung gegeben wird.

Die für die erfindungsgemäßen Verbindungen ermittelten Hemmwerte ergeben sich aus der folgenden Tabelle A, in der die Nummern der Verbindungen den Nummern der Beispiele entsprechen.

Tabelle A

| Hemmung der PDE IV-Aktivität | |
|---|---|
| **Verbindung** | **-log $IC_{50}$** |
| 1 | 7,39 |
| 2 | 8,84 |
| 3 | 7,73 |
| 4 | 8,73 |

Tabelle A (fortgesetzt)

| Hemmung der PDE IV-Aktivität | |
|---|---|
| **Verbindung** | **-log $IC_{50}$** |
| 5 | 7,02 |
| 6 | 8.14 |
| 7 | 6,34 |
| 8 | 5,92 |
| 9 | 8,52 |
| 10 | 7,48 |
| 11 | 7,52 |
| 12 | 4,95 |
| 13 | 7,39 |
| 14 | 8,27 |
| 15 | 6,81 |
| 16 | 8,66 |
| 17 | 6,79 |
| 18 | 7,69 |
| 19 | 7,55 |
| 20 | 5,18 |

## Patentansprüche

**1.** Verbindungen der Formel I,

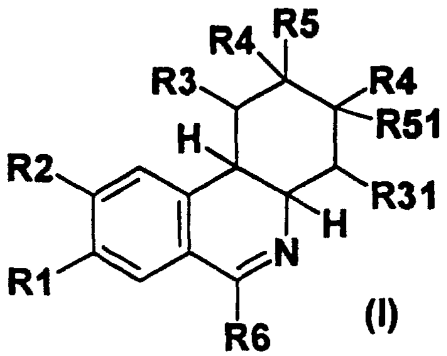

(I)

worin

R1 Hydroxy, 1-4C-Alkoxy, 3-7C-Cycloalkoxy oder 3-7C-Cycloalkylmethoxy oder ganz oder teilweise durch Fluor substituiertes 1-4C-Alkoxy bedeutet,
R2 Hydroxy, 1-4C-Alkoxy, 3-7C-Cycloalkoxy oder 3-7C-Cycloalkylmethoxy oder ganz oder teilweise durch Fluor substituiertes 1-4C-Alkoxy bedeutet,

oder worin

R1 und R2 gemeinsam eine 1-2C-Alkylendioxygruppe bedeuten,

R3 Wasserstoff oder 1-4C-Alkyl bedeutet,
R31 Wasserstoff oder 1-4C-Alkyl bedeutet,

oder worin

R3 und R31 gemeinsam eine 1-4C-Alkylengruppe bedeuten,
R4 Wasserstoff oder 1-4C-Alkyl bedeutet,
R5 Wasserstoff bedeutet,
R51 Wasserstoff bedeutet,

oder worin

R5 und R51 gemeinsam eine zusätzliche Bindung darstellen,
R6 einen durch R7 substituierten Phenylrest darstellt, wobei
R7 COOR71 oder CON(R72)R73 bedeutet und

R71 Wasserstoff, 1-7C-Alkyl, 3-7C-Cycloalkyl oder 3-7C-Cycloalkylmethyl bedeutet und
R72 und R73 unabhängig voneinander

Wasserstoff, 1-7C-Alkyl, 3-7C-Cycloalkyl oder 3-7C-Cycloalkylmethyl bedeuten, sowie die Salze dieser Verbindungen.

**2.** Verbindungen der Formel I nach Anspruch 1, in denen

R1 1-4C-Alkoxy, 3-7C-Cycloalkoxy oder 3-7C-Cycloalkylmethoxy oder ganz oder teilweise durch Fluor substituiertes 1-2C-Alkoxy bedeutet,
R2 1-4C-Alkoxy, 3-7C-Cycloalkoxy oder 3-7C-Cycloalkylmethoxy oder ganz oder teilweise durch Fluor substituiertes 1-2C-Alkoxy bedeutet,
R3 Wasserstoff bedeutet,
R31 Wasserstoff bedeutet,

oder worin

R3 und R31 gemeinsam eine 1-2C-Alkylengruppe bedeuten,
R4 Wasserstoff oder 1-4C-Alkyl bedeutet,
R5 Wasserstoff bedeutet,
R51 Wasserstoff bedeutet,

oder worin

R5 und R51 gemeinsam eine zusätzliche Bindung darstellen,
R6 einen durch R7 substituierten Phenylrest darstellt, wobei
R7 COOR71 oder CON(R72)R73 bedeutet und

R71 Wasserstoff, 1-7C-Alkyl oder 3-7C-Cycloalkylmethyl bedeutet und
R72 und R73 unabhängig voneinander Wasserstoff oder 1-7C-Alkyl bedeuten,

sowie die Salze dieser Verbindungen.

**3.** Verbindungen der Formel I nach Anspruch 1, in denen

R1 1-4C-Alkoxy, 3-7C-Cycloalkoxy oder ganz oder teilweise durch Fluor substituiertes 1-2C-Alkoxy bedeutet,
R2 1-4C-Alkoxy, 3-7C-Cycloalkoxy oder ganz oder teilweise durch Fluor substituiertes 1-2C-Alkoxy bedeutet,
R3 Wasserstoff bedeutet,
R31 Wasserstoff bedeutet,

oder worin

| | |
|---|---|
| R3 und R31 | gemeinsam eine 1-2C-Alkylengruppe bedeuten, |
| R4 | Wasserstoff oder 1-4C-Alkyl bedeutet, |
| R5 | Wasserstoff bedeutet, |
| R51 | Wasserstoff bedeutet, |

oder worin

| | |
|---|---|
| R5 und R51 | gemeinsam eine zusätzliche Bindung darstellen, |
| R6 | einen durch R7 substituierten Phenylrest darstellt, wobei |
| R7 | COOR71 oder CON(R72)R73 bedeutet und |

| | |
|---|---|
| R71 | Wasserstoff, 1-7C-Alkyl oder 3-7C-Cycloalkylmethyl bedeutet und |
| R72 und R73 | unabhängig voneinander Wasserstoff oder 1-4C-Alkyl bedeuten, |

sowie die Salze dieser Verbindungen.

**4.** Verbindungen der Formel I nach Anspruch 1, in denen

| | |
|---|---|
| R1 | Methoxy, Ethoxy oder Difluormethoxy bedeutet, |
| R2 | Methoxy, Ethoxy oder Difluormethoxy bedeutet, |
| R3 und R31 | Wasserstoff bedeuten, |
| R4 | Wasserstoff bedeutet, |
| R5 und R51 | Wasserstoff bedeuten, |
| R6 | einen durch R7 substituierten Phenylrest darstellt, wobei |
| R7 | COOR71 oder CON(R72)R73 bedeutet und |

| | |
|---|---|
| R71 | Wasserstoff oder 1-4C-Alkyl bedeutet, |
| R72 | Wasserstoff bedeutet und |
| R73 | 1-4C-Alkyl bedeutet; |

sowie die Salze dieser Verbindungen.

**5.** Arzneimittel enthaltend eine oder mehrere Verbindungen nach Anspruch 1 zusammen mit pharmazeutischen Hilfs- und/oder Trägerstoffen.

**6.** Verwendung von Verbindungen nach Anspruch 1 zur Herstellung von Arzneimitteln für die Behandlung von Atemwegserkrankungen.

## Claims

**1.** Compounds of the formula I

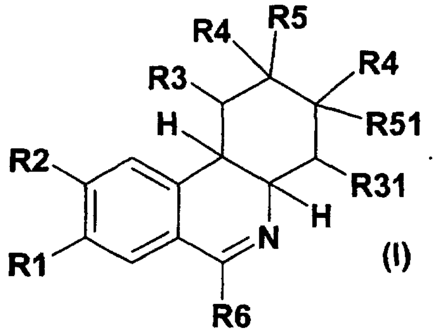

(I)

in which

R1 is hydroxy, 1-4C-alkoxy, 3-7C-cycloalkoxy or 3-7C-cycloalkylmethoxy or 1-4C-alkoxy which is completely or partially substituted by fluorine,
R2 is hydroxy, 1-4C-alkoxy, 3-7C-cycloalkoxy or 3-7C-cycloalkylmethoxy or 1-4C-alkoxy which is completely or partially substituted by fluorine,

or in which

R1 and R2 together are a 1-2C-alkylenedioxy group,
R3 is hydrogen or 1-4C-alkyl,
R31 is hydrogen or 1-4C-alkyl,

or in which

R3 and R31 together are a 1-4C-alkylene group,
R4 is hydrogen or 1-4C-alkyl,
R5 is hydrogen
R51 is hydrogen,

or in which

R5 and R51 together are an additional bond,
R6 is a phenyl radical which is substituted by R7, where
R7 is COOR71 or CON(R72)R73 and

R71 is hydrogen, 1-7C-alkyl, 3-7C-cycloalkyl or 3-7C-cycloalkylmethyl and
R72 and R73 independently of one another are each hydrogen, 1-7C-alkyl, 3-7C-cycloalkyl or 3-7C-cycloalkylmethyl,

and the salts of these compounds.

**2.** Compounds of the formula I as claimed in claim 1, in which

R1 is 1-4C-alkoxy, 3-7C-cycloalkoxy or 3-7C-cycloalkylmethoxy or 1-2C-alkoxy which is completely or partially substituted by fluorine,
R2 is 1-4C-alkoxy, 3-7C-cycloalkoxy or 3-7C-cycloalkylmethoxy or 1-2C-alkoxy which is completely or partially substituted by fluorine,
R3 is hydrogen,
R31 is hydrogen,

or in which

R3 and R31 together are a 1-2C-alkylene group,
R4 is hydrogen or 1-4C-alkyl,
R5 is hydrogen,
R51 is hydrogen,

or in which

R5 and R51 together are an additional bond,
R6 is a phenyl radical which is substituted by R7, where
R7 is COOR71 or CON(R72)R73 and

R71 is hydrogen, 1-7C-alkyl or 3-7C-cycloalkylmethyl and
R72 and R73 independently of one another are each hydrogen or 1-7C-alkyl,

and the salts of these compounds.

**3.** Compounds of the formula I as claimed in claim 1, in which

R1 is 1-4C-alkoxy, 3-7C-cycloalkoxy or 1-2C-alkoxy which is completely or partially substituted by fluorine,
R2 is 1-4C-alkoxy, 3-7C-cycloalkoxy or 1-2C-alkoxy which is completely or partially substituted by fluorine,
R3 is hydrogen,
R31 is hydrogen,

or in which

R3 and R31 together are a 1-2C-alkylene group,
R4 is hydrogen or 1-4C-alkyl,
R5 is hydrogen,
R51 is hydrogen,

or in which

R5 and R51 together are an additional bond,
R6 is a phenyl radical which is substituted by R7, where
R7 is COOR71 or CON(R72)R73 and

R71 is hydrogen, 1-7C-alkyl or 3-7C-cycloalkylmethyl and
R72 and R73 independently of one another are each hydrogen or 1-4C-alkyl,

and the salts of these compounds.

**4.** Compounds of the formula I as claimed in claim 1, in which

R1 is methoxy, ethoxy or difluoromethoxy,
R2 is methoxy, ethoxy or difluoromethoxy,
R3 and R31 are each hydrogen,
R4 is hydrogen,
R5 and R51 are each hydrogen,
R6 is a phenyl radical which is substituted by R7, where
R7 is COOR71 or CON(R72)R73 and

R71 is hydrogen or 1-4C-alkyl,
R72 is hydrogen and
R73 is 1-4C-alkyl,

and the salts of these compounds.

**5.** A medicament comprising one or more compounds as claimed in claim 1 together with pharmaceutical auxiliaries and/or excipients.

**6.** The use of compounds as claimed in claim 1 for the production of medicaments for the treatment of respiratory diseases.

**Revendications**

**1.** Composés de formule I

(I)

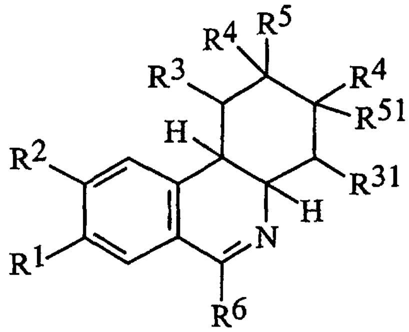

dans laquelle

- $R^1$ représente un groupe hydroxy, alcoxy en $C_{1-4}$, cycloalcoxy en $C_{3-7}$ ou (cycloalkyle en $C_{3-7}$)méthoxy ou un groupe alcoxy en $C_{1-4}$ partiellement ou complètement fluoré,
- $R^2$ représente un groupe hydroxy, alcoxy en $C_{1-4}$, cycloalcoxy en $C_{3-7}$ ou (cycloalkyle en $C_{3-7}$)méthoxy ou un groupe alcoxy en $C_{1-4}$ partiellement ou complètement fluoré, ou
- $R^1$ et $R^2$ représentent, ensemble, un groupe alkylènedioxy en $C_{1-2}$,
- $R^3$ représente un atome d'hydrogène ou un groupe alkyle en $C_{1-4}$,
- $R^{31}$ représente un atome d'hydrogène ou un groupe alkyle en $C_{1-4}$, ou
- $R^3$ et $R^{31}$ représentent, ensemble, un groupe alkylène en $C_{1-4}$,
- $R^4$ représente un atome d'hydrogène ou un groupe alkyle en $C_{1-4}$,
- $R^5$ représente un atome d'hydrogène,
- $R^{51}$ représente un atome d'hydrogène, ou
- $R^5$ et $R^{51}$ représentent, ensemble, une liaison supplémentaire,
- $R^6$ représente un résidu phényle portant un substituant $R^7$,
- $R^7$ représente un groupe $COOR^{71}$ ou $CON(R^{72})R^{73}$, où
- $R^{71}$ représente un atome d'hydrogène, un groupe alkyle en $C_{1-7}$, cycloalkyle en $C_{3-7}$ ou (cycloalkyle en $C_{3-7}$)méthyle et
- $R^{72}$ et $R^{73}$ représentent chacun, indépendamment, un atome d'hydrogène, un groupe alkyle en $C_{1-7}$, cycloalkyle en $C_{3-7}$ ou (cycloalkyle en $C_{3-7}$)méthyle,

ainsi que les sels de ces composés.

**2.** Composés de formule (I) selon la revendication 1, dans laquelle

- $R^1$ représente un groupe alcoxy en $C_{1-4}$, cycloalcoxy en $C_{3-7}$ ou (cycloalkyle en $C_{3-7}$)méthoxy ou un groupe alcoxy en $C_{1-2}$ partiellement ou complètement fluoré,
- $R^2$ représente un groupe alcoxy en $C_{1-4}$, cycloalcoxy en $C_{3-7}$ ou (cycloalkyle en $C_{3-7}$)méthoxy ou un groupe alcoxy en $C_{1-2}$ partiellement ou complètement fluoré,
- $R^3$ représente un atome d'hydrogène,
- $R^{31}$ représente un atome d'hydrogène, ou
- $R^3$ et $R^{31}$ représentent, ensemble, un groupe alkylène en $C_{1-2}$,
- $R^4$ représente un atome d'hydrogène ou un groupe alkyle en $C_{1-4}$,
- $R^5$ représente un atome d'hydrogène,
- $R^{51}$ représente un atome d'hydrogène, ou
- $R^5$ et $R^{51}$ représentent, ensemble, une liaison supplémentaire,
- $R^6$ représente un résidu phényle portant un substituant $R^7$,
- $R^7$ représente un groupe $COOR^{71}$ ou $CON(R^{72})R^{73}$, où
- $R^{71}$ représente un atome d'hydrogène, un groupe alkyle en $C_{1-7}$ ou (cycloalkyle en $C_{3-7}$)méthyle et
- $R^{72}$ et $R^{73}$ représentent chacun, indépendamment, un atome d'hydrogène ou un groupe alkyle en $C_{1-7}$,

ainsi que les sels de ces composés.

**3.** Composés de formule (I) selon la revendication 1, dans laquelle

$R^1$ représente un groupe alcoxy en $C_{1-4}$, cycloalcoxy en $C_{3-7}$ ou alcoxy en $C_{1-2}$ partiellement ou complètement fluoré,
$R^2$ représente un groupe alcoxy en $C_{1-4}$, cycloalcoxy en $C_{3-7}$ ou alcoxy en $C_{1-2}$ partiellement ou complètement fluoré,
$R^3$ représente un atome d'hydrogène,
$R^{31}$ représente un atome d'hydrogène, ou
$R^3$ et $R^{31}$ représentent, ensemble, un groupe alkylène en $C_{1-2}$,
$R^4$ représente un atome d'hydrogène ou un groupe alkyle en $C_{1-4}$,
$R^5$ représente un atome d'hydrogène,
$R^{51}$ représente un atome d'hydrogène, ou
$R^5$ et $R^{51}$ représentent, ensemble, une liaison supplémentaire,
$R^6$ représente un résidu phényle portant un substituant $R^7$,
$R^7$ représente un groupe $COOR^{71}$ ou $CON(R^{72})R^{73}$, où
$R^{71}$ représente un atome d'hydrogène, un groupe alkyle en $C_{1-7}$ ou (cycloalkyle en $C_{3-7}$)méthyle et
$R^{72}$ et $R^{73}$ représentent chacun, indépendamment, un atome d'hydrogène ou un groupe alkyle en $C_{1-4}$,

ainsi que les sels de ces composés.

**4.** Composés de formule (I) selon la revendication 1, dans laquelle

$R^1$ représente un groupe méthoxy, éthoxy ou difluorométhoxy,
$R^2$ représente un groupe méthoxy, éthoxy ou difluorométhoxy,
$R^3$ et $R^{31}$ représentent chacun un atome d'hydrogène,
$R^4$ représente un atome d'hydrogène,
$R^5$ et $R^{51}$ représentent chacun un atome d'hydrogène,
$R^6$ représente un résidu phényle portant un substituant $R^7$, où
$R^7$ représente un groupe $COOR^{71}$ ou $CON(R^{72})R^{73}$ et
$R^{71}$ représente un atome d'hydrogène ou un groupe alkyle en $C_{1-4}$,
$R^{72}$ représente un atome d'hydrogène et
$R^{73}$ un groupe alkyle en $C_{1-4}$,

ainsi que les sels de ces composés.

**5.** Médicament contenant un ou plusieurs composés selon la revendication 1 ainsi que des adjuvants et/ou des excipients pharmaceutiques.

**6.** Utilisation de composés selon la revendication 1, pour la fabrication de médicaments destinés au traitement de maladies des voies respiratoires.

FORMELBLATT

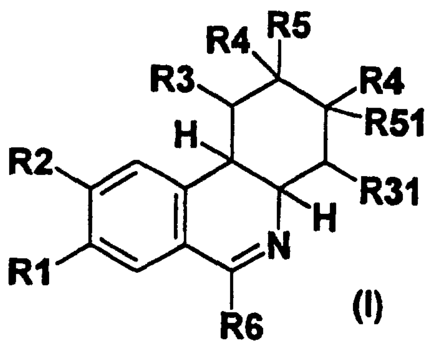

(I)

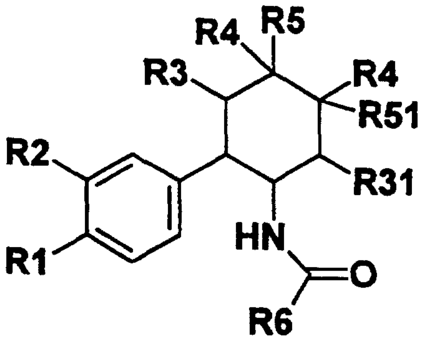

(II)

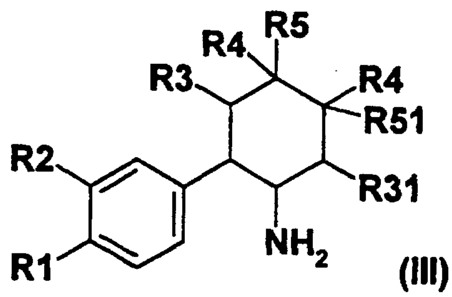

(III)

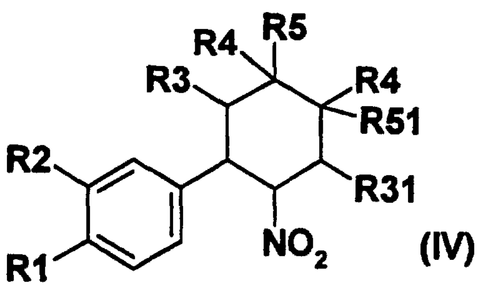

(IV)

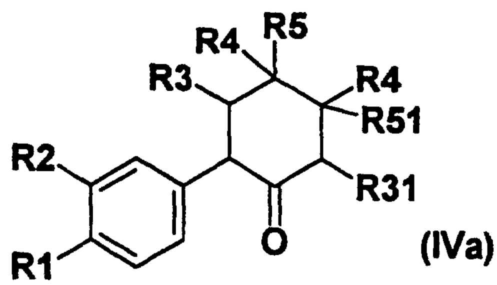

(IVa)

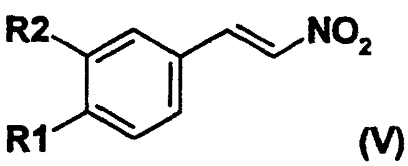

(V)

R3-CH=C(R4)-C(R4)=CH-R31 (VI)

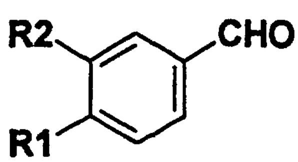

(VII)